Europäisches Patentamt

European Patent Office

Office européen des brevets

(11) Publication number: **0 405 273**
**A2**

# EUROPEAN PATENT APPLICATION

(21) Application number: **90111409.0**

(22) Date of filing: **17.06.90**

(51) Int. Cl.⁵: **C07K 5/06**

(30) Priority: **26.06.89 JP 160960/89**

(43) Date of publication of application:
**02.01.91 Bulletin 91/01**

(84) Designated Contracting States:
**DE ES FR GB IT NL**

(71) Applicant: **MITSUI TOATSU CHEMICALS,
INCORPORATED**
**2-5, 3-chome, Kasumigaseki
Chiyoda-ku Tokyo(JP)**

(72) Inventor: **Ajioka, Masanobu**
**1172-3 Kamikuratacho, Totsuka-ku
Yokohama-shi, Kanagawa-ken(JP)**
Inventor: **Higuchi, Chojiro**
**4-5-13 Dai
Kamakura-shi, Kanagawa-ken(JP)**
Inventor: **Oura, Takeshi**
**4-10-8 Hisagi
Zushi-shi, Kanagawa-ken(JP)**
Inventor: **Katoh, Toshio**
**600-1-5-536 Kamisakunobe, Takatsu-ku
Kawasaki-shi, Kanagawa-ken(JP)**
Inventor: **Yamaguchi, Akihiro**
**1-13-24 Zaimokuza
Kamakura-shi, Kanagawa-ken(JP)**

(74) Representative: **Schüler, Horst, Dr.**
**Kaiserstrasse 69
D-6000 Frankfurt/Main 1(DE)**

(54) Method for producing readily soluble crystalline alfa-L-aspartyl-L-phenylalanine methyl ester.

(57) A method for producing a readily soluble crystalline form of α-L-aspartyl-L-phenylalanine methyl ester (α-APM) which comprises adding a solution of α-APM in an aqueous solvent to a stirred suspension of α-APM while maintaining the latter at a temperature lower than the solution.

# METHOD FOR PRODUCING READILY SOLUBLE CRYSTALLINE α-L-ASPARTYL-L-PHENYLALANINE METH-YL ESTER

## BACKGROUND OF THE INVENTION

### (a) Field of the Invention

The present invention relates to a method for producing crystalline α-L-aspartyl-L-phenylalanien emthyl ester (hereinafter abbreviated as "α-APM") which is readily soluble in water, i.e., which has improved solubilization.

### (b) Description of the Prior Art

α-APM is widely known as a dipeptide base sweetener. It has sweetness of good quality and a degree of sweetness about 200 times the sweetness of sucrose. The demand for α-APM as a diet sweetener is rapidly expanding.

α-APM is a dipeptide compound composed of L-aspartic acid and L-phenylalanine methyl ester. It can be prepared by both chemical and biochemical processes, the latter utilizing microorganisms. Various methods have been disclosed for each process.

As a typical example of a chemical process of producing α-APM, L-aspartic anhydride having a protected amino group is subjected to a condensation reaction with L-phenylalanine methyl ester in a suitable solvent and subsequently cleaving the protective group by a conventional method to obtain α-APM (for example, U.S. Patent 3,786,039). In a representative biochemical process, N-benzyloxycarbonyl-L-aspartic acid and L-phenylalanine methyl ester are condensed in the presence of metalloprotease to obtain N-benzyloxycarbonyl-α-L-asparltyl-L-phenylalanine methyl ester. The benzyloxycarbonyl group is then removed from the intermediate by catalytic reduction to give α-APM.

In the industrial production of α-APM, a step of purifying the crude α-APM obtained as the final product, by isolating α-APM from the reaction mass, is inevitable, whichever of the above mentioned processes is employed. The purification step is usually carried out by recrystallization from a solution of water or a water-containing solvent (water or a water-containing solvent is hereinafter referred to as aqueous medium). Another method for removing impurities involves a stirring treatment of a suspension of α-APM in an aqueous medium, depending upon the quality of crude α-APM. However, pure α-APM obtained by this purification method forms a hard block in the dry state. Therefore, crushing is required for the preparation of final product, which causes difficulties in the handling of α-APM. Also, a long period of time is required for the drying and the content of 5- benzyl-3,6-dioxo-2-piperazine acetic acid (DKP), which is an intramolecular cyclization product of α-APM therein is liable to increase. Thus the method causes problems in the manufacture of product having uniform quality.

α-APM purified y conventional recrystallization methods has the disadvantage of poor solubilization (rate of solution) in water. For example, when α-APM obtained conventionally by recrystallization from a 50 vol.% aqueous methanol solution is crushed and 250 mg of the resultant powder is poured into 250 ml of water with stirring at the room temperature and the solubilization thereof is determined by alternatively and repeatedly stirring and standing every 30 seconds, a significant amount of undissolved α-APM remains even after 5 minutes and more than 15 minutes is required for it to dissolve completely. α-APM obtained by recrystallization from water also has similar low solubilization.

Because the present demand for α-APM as a sweetener is primarily in the field of soft drinks, the solubilization of α-APM in water is definitely an important factor in the determination of product specifications. However, there is little prior art relating to improving the solubilization of α-APM itself.

Japanese Laid-Open patent No. 177952/1983 discloses a method for crystallizing α-APM from an aqueous solution in which the initial concentration of α-APM is 2-10 wt.% and the aqueous solution is cooled with control of heat conduction and without providing forced flow, such as with mechanical stirring. The total mass is converted to a pseudo-solid phase having the consistency of a sherbet and, if necessary, furhter cooled. The thus-produced crystals of α-APM have enhanced filterability and the improved properties of fine particles, such as bulk density and the like. The α-APM thus-obtained is described as having excellent solubilization as compared to conventionally crystallized product. Although this method certainly improves various properties of the fine particles of α-APM thus. isolated, including solubilization to a remarkable extent, because the crystallization from the aqueous solution is carried out by cooling without forced flow, such as mechanical stirring, conventional crystallizing equipment requires a remarkably long time for the cooling process to commplete. This requires an increase in equipment scale and restricts its industrial application. Therefore, the patent specifies the maximum distance between the cooling surface and cooling zone and proposes that a special crystallizaltion device be employed to meet this requirement. Thus, as a practical matter, the method of Japanese Laid-Open Patent No. 177952/1983 cannot be employed industrially without using the special crystallization device described therein.

SUMMARY OF THE INVENTION

An object of the present invention is to provide a method for preparing readily soluble crystalline α-APM having improved solubilization, i.e., an improved rate of dissolution in water at room temperature, which can be achieved by conventional techniques.

This object can be accomplished by crystallizing α-APM from an aqueous α-APM solution which comprises the step of adding a solution of α-APM in an aqueous solvent to a stirred suspension of α-APM which is maintained at a temperature lower than the solution.

In this method, the purification technique employed is simple and operation is also easier than the above-mentioned prior art, and the crystals of α-APM which can thus be obtained thereby are excellent in solubilizations, i.e., they have an improved rate of dissolution in water at room temperature. In short, the present invention is industrially advantageous.

DETAILED DESCRIPTION OF THE INVENTION

The present inventors have found that the problems of the above-mentioned conventional techniques are avoided or eliminated when a solution of α-APM in an aqueous solvent is added to a suspension which has a temperature lower than the aqueous solution and in which crystals of α-APM are suspended, while the suspension is stirred, so that crystals of α-APM from the solution are deposited in the suspension. The thus deposited crystals do not adhere to a cooling surface, in contrast to the case where crystals are rapidly deposited from a super-saturated solution. In addition, the α-APM crystals obtained by solid-liquid separation and then drying are in the form of large crystals which have excellent filtering characteristics, and solubilization in water. Thus, the present invention has been completed on the basis of this knowledge.

The present invention is a method for producing a readily soluble crystalline form of α-APM by precipitating α-APM from an aqueous solution thereof into a stirred suspension of α-APM in an aqueous solvent which is maintained at a temperature lower than the solution.

The α-APM employed in the method of the present invention can be prepared by various methods without restriction.

The α-APM in the suspension is preferably pure, such as food grade, e.g., contains no more than about 2% impurities, such as β-APM and 5-benzyl-3,6-dioxo-2-piperazine acetic acid (DKP). The α-APM in the starting solution can be pure, i.e., the process can be used to convert pure powdered α-APM with poor solubilization into a crystalline form having improved rate of dissolution in water. The process can be used to concurrently purify impure α-APM, e.g., of purity of less than 90%, for example, α-APM whose predominant impurity is DKP and/or which had poor solubilization, i.e., which does not fully dissolve at room temeprature in an equal weight of water within 15 minutes, even with repeated stirring and standing every 30 seconds.

Examples of the aqueous solvent used in the method of the present invention include water and a mixture of water and a lower alcohol such as methanol, ethanol, isopropanol or tert-butanol. Other organic solvents which are miscible with water can also be used, so long as they do not impair the object of the present invention. When an aqueous medium containing a lower alcohol is used, the concentration of the lower alcohol therein is preferably 60% by weight or less.

The concentration of α-APM in the aqueous α-APM solution is usually 60% or more of saturation concentration and preferably is near, e.g., 80% or higher, the saturation concentration at the solution temperature.

On the other hand, the concentration of α-APM in the α-APM suspension is not limited but should be such that α-APM remains in the state of the suspension at a temperature thereof.

The weight ratio of α-APM in the solution to the α-APM in the suspension is usually from about 2:1 to about 10:1, preferably about 4:1, i.e., a substantially larger amount of α-APM is in the starting solution than in the starting suspension.

The temperature of the starting aqueous α-APM solution ordinarily is in the range of from 20 to 70°C, preferably from 30 to 60°C. When this temperature is less than 20°C, the efficiency of the crystallization is poor owing to the low solubility, and conversely when it is more than 70°C, dissolved α-APM undesirably thermally decomposes to produce DKP and α-L-aspartyl-L-phenylalanine.

The temperature of the suspension is required to be lower than that of the α-APM solution to be added, and a temperature difference therebetween is from 10 to 70°C, preferably 40 to 70°C. The higher the volume ratio of solution solvent to suspension solvent, the larger the temperature differential should be. Preferably, the suspension is maintained at a temperature below room temperature throughout the addition of the solution thereto, e.g., at -10°C to 15°C, preferably about 5°C.

In the present invention, the solution of α-APM is added to the suspension of α-APM with thorough mixing which ensures substantially instantaneous cooling of the added solution to the temperature of the suspension, e.g., by blades of a stirrer or foced convection by blowing a stream of air bubbles through the suspension or like means which rapidly and uniformly mixes the above- mentioned α-APM solution with the suspension and effectively cool the resultant mixture, which will be described hereinafter.

As the suspension having a lower temperature is mixed with the warmer aqueous α-APM solution, the temperature of the resulting mixture rises gradually. Therefore, to light or eliminate this effect, it is necessary that the mixture is cooled so that the temperature of the mixture is kept within a predetermined range, preferably a temperature equal to or up to 5°C higher than that of the starting suspension. This temperature can be maintained by adjusting the feed rate of the above-mentioned aqueous solution and the cooling rate.

The cooling of the mixture can be achieved by usual means, for example, a cooling coil which cools the outer surface of the container in which the mixing operation is carried out.

In the practice of the method of the present invention, the starting α-APM solution can be obtained by dissolving hard to dissolve impure or pure α-APM in a high-temperature aqueous solvent, viz., one which produces a solution of α-APM having a temperature from 20-70°C or by heating an aqueous solution of α-APM produced in situ , e.g., by treating a salt thereof with a base. The starting suspension is prepared by slowly cooling a part of the resulting solution, or by dispersing α-APM crystals in an aqueous medium and being cooled. The above-mentioned high-temperature α-APM solution is added to the cooler suspension. While the solution is being added, the temeprature of the suspension is maintained at a predetermined level as described above by adjusting the feed rate of the solution and/or a cooling rate.

The high-temperature α-APM solution which has been added to the low-temperature suspension is rapidly cooled, thereby precipitating crystals of α-APM from the solution. The precipitated crystals do not adhere to the cooling surface of a cooling means for the mixture, which is convenient for handling the thus deposited crystals. The latter are isolated by filtration and then dried, thereby obtaining α-APM which has excellent solubilization.

The present invention will be described in detail in the following examples, in which the solubilization test was carried out by the following procedure: Solubilization Test

Into 250 ml of pure water having a temperature of 26 ± 2°C which was being stirred (at 350 to 360 rpm by a magnetic stirrer) was poured 250 mg of a ground α-APM sample. Stirring and standing were repeated every 30 seconds. The time required for the sample to dissolve completely was measured.

Example 1

In a 1-liter flask were placed 141.1 g of water and 5.9 g of an α-APM powder (100% purity) in order to prepare a suspension. The temperature of the suspension was maintained at 5°C by cooling the outside of the flask with ice water, while it was stirred with a stirrer. A solution of α-APM at 60°C, prepared by dissolving 24.0 g of crude α-APM containing 0.5 g of DkP in 564.5 g of water, was added dropwise over a 20 minutes period to the above-mentioned stirred suspension to give 735.5 g of a suspension mother liquor. During the addition, the contents of the flask was stirred with a stirrer and the temperature thereof was manufactured at about 5°C. Adhesion of crystals to the wall of the flask was not ob served. After completion of the addition, the deposited crystals were filtered through a Kiriyama funnel having a diameter of 60 mm under reduced pressure of 30 mmHg, and after 20 minutes, 46.0 g of a wet cake (water content 44%) was obtained. The yield of the product to the total α-APM was 87.6%, and according to the results of HPLC analysis, the DKP content of the α-APM was 0.1%.

This cake was then dried with warm air of 50 to 60°C. When the dried crystals were then subjected to the above-describe solubilization test, the dried crystals were completely dissolved within 5 minutes.

Example 2

In a 1-liter flask, 30.1 g of crude α-APM containing 0.7 g of DKP was dissolved in 705.6 g of water at 60°C, and 20% of the resulting solution was placed in another 1-liter flask and then cooled to 5°C over 2 hours, thereby precipitating crystals of α-APM therefrom. The remaining α-APM solution was added dropwise to the suspension over 20 minutes, while the suspension was maintained at 5°C and stirred with a stirrer. During the addition, the precipitated solids in the flask were maintained as a uniform suspension by the stirring. No adhesion of crystals to the wall of the flask was observed. After completion of the addition, the deposited crystals were filtered through a Kiriyama funnel having a diameter of 60 mm under reduced pressure of 30 mmHg, and after 20 minutes, 47.8 g of wet cake (water content 46.6%) was obtained. The yield of α-APM based on the starting α-APM was 86.8%, and according to the results of HPLC analysis, the DKP content of the recovered α-APM was 0.1%.

This cake was then dried with warm air at 50 to 60°C. In the solubilization test, the dried crystals were completely dissolved within 5 minutes.

Comparative Example

In a 1-liter flask, 30.1 g of crude α-APM containing 0.7 g of DKP was dissolved in 705.6 g of water at 60°C and while being stirred, the temperature thereof was lowered by cooling the outside of the flask with water at 25°C. As the temperature in the flask dropped, crystals of precipitated α-APM adhered to the wall of the flask, so that the rate of temperature drop was reduced. When the temperature in the flask had reached 35°C after 20 minutes, ice water was then used to cool the outside of the flask. 30 minutes were required to cool the suspension in the flask to 5°C. The deposited crystals were then filtered through a Kiriyama funnel having a diameter of 60 mm under reduced pressure of 30 mmHg, and after 20 minutes, 79.6 g of wet cake (water content 67.5%) was obtained. The yield was 88.0%. According to the results of HPLC analysis, the content of DKP to α-APM was 0.2%.

This cake was then dried with warm air of 50 to 60°C. A portion of resulting crystals, when subjected to the solubilization test, required 25 minutes to be completely dissolveld.

Example 3

Following the same procedure as in Example 1, 588.3 g of the 735.5 g of the suspension mother liquid thus obtained was filtered through a Kiriyama funnel having a diameter of 60 mm under reduced pressure of 30 mmHg, and after 20 minutes, 34.5 g of wet cake (water content 42%) was obtained. The yield of the product to the total α-APM was 68.1%, and according to the results of HPLC analysis, the content of DKP was 0.1%.

Next, while maintaining 147.2 g of the thus-obtained suspension at 5°C with stirring provided by a stirrer, a solution at 60°C prepared by dissolving 24.0 g of crude α-APM containing 0.5 g of DKP in 564.5 g of water was added dropwise to the above-mentioned suspension over 20 minutes. During the addition, the stirring state of the suspension was good, and adhesion of crystals to the wall of the flask was not observed. After completion of the addition, the precipitated crystals were filtered through the Kiriyama funnel having a diameter of 60 mm under reduced pressure of 30 mmHg, and after 20 minutes, 44.4 g of wet cake (water content 41%) was obtained. The dry weight yield of product based on the α-APM in solution used in the adding step was 111.5%. According to the results of HPLC analysis, the DKP content thereof was 0.1%. The total yield to the total used α-APM was 79.6%.

This cake was then dried with warm air of 50 to 60°C. The resulting crystals, when subjected to the solubilization test, completely dissolved within 5 minutes.

Example 4

In a 1-liter flask were placed a mixture of 33.9 g of methanol, 33.9 g of water and 5.9 g of an α-APM powder (100% purity) in order to prepare a suspension. The temperature of the suspension was maintained at 5°C by cooling the outside of the flask with ice water, while stirring the suspension with a stirrer. A solution prepared by dissolving 24.0 g of crude α-APM containing 0.5 g of DKP in 138.0 g of methanol and 138.0 g of water at 50°C was added dropwise to the stirred and cooled suspension over 20 minutes. During the addition, the suspension was well stirred, and no adhesion of crystals to the wall of the flask was observed. After completion of the addition, the deposited crystals were filtered through a kiriyama funnel having a diameter of 60 mm under reduced pressure of 30 mmHg, and after 20 minutes, ·38.4 g of wet cake (liquid content 44%) was obtained. The recovering of α-APM based on the total starting α-APM was 87.2%. According to the results of HPLC analysis, the DKP content of the α-APM was 0.2%.

This cake was then dried with warm air to 50 to 60°C. When subjected to the solubilization test, the dried crystals were completely dissolved within 5 minutes.

Example 5

In a 1-liter flask were placed 46.2 g of ethanol, 46.2 g of water and 5.9 g of an α-APM powder to obtain a suspension. The temperature of the suspension was maintained at 5°C by cooling the outside of the flask with ice water while it was stirred with a stirrer, a solution prepared by dissolving 24.0 g of crude α-APM containing 0.5 g of DKP in 188.0 g of methanol and 188.0 g of water at 50°C was added dropwise to the cooled and stirred suspension over a 20 minutes period. During the addition, the suspension was well stirred and no adhesion of crystals to the wall of the flask was not observed. After completion of the addition, the deposited crystals were filtered through a Kiriyama funnel having a diameter of 60 mm under reduced pressure of 30 mmHg, and after 20 minutes, 42.5 g of wet cake (liquid content 37.5%) was obtained. The recovery of the $\alpha$-APM based on the total starting $\alpha$-APM was 90.3%. According to the results of HPLC analysis, the DKP content of the $\alpha$-APM was 0.2%.

This cake was then dried with warm air of 50 to 60°C. When the resulting crystals were subjected to the solubilization test, the dried crystals were completely dissolved within 5 minutes.

## Claims

1. A method for producing readily soluble crystalline $\alpha$-L-aspartyl-L-phenylalanine methyl ester ($\alpha$-APM) which comprises the step of adding a solution of $\alpha$-APM in an aqueous solvent to a stirred suspension of substantially pure $\alpha$-APM in an aqueous solvent while maintaining the suspension at a temperature lower than said solution.

2. A method according to Claim 1 wherein the temperature of said suspension is about 40 to 70°C lower than that of said solution added thereto.

3. A method according to Claim 1 wherein the temperature of the solution is from 30 to 60°C.

4. A method according to Claim 1 wherein the temperature of the suspension is maintained at about 5°C.

5. A method according to Claim 1 wherein the concentration of the $\alpha$-APM in solution is at or near saturation.

6. A method according to Claim 1 wherein the amount of $\alpha$-APM in the solution is substantially larger than the amount in the suspension.

7. A method according to Claim 1 wherein the $\alpha$-APM in the suspension is substantially pure and in the form of a powder.

8. A method according to Claim 1 wherein the aqueous solvent in both the solution and in the suspension is water or a mixture of water and methanol.

9. A method according to Claim 1, wherein the temperature of said suspension is about 40 to 70°C lower than that of said solution added thereto; wherein the temperature of the solution is from 30 to 60°C; wherein the temperature of the suspension is maintained at about 5°C; wherein the concentration of the $\alpha$-APM in solution is at or near saturation; wherein the amount of $\alpha$-APM in the solution is substantially larger than the amount in the suspension; wherein the $\alpha$-APM in the suspension is substantially pure and in the form of a powder; and wherein the aqueous solvent in both the solution and in the suspension is water or a mixture of water and methanol.